# EUROPEAN PATENT APPLICATION

(11) **EP 1 241 268 A2**
(43) Date of publication of application: **18.09.2002**
(21) Application number: 02251643.9
(22) Date of filing: 08.03.2002
(51) Int. Cl.: C12Q 1/68

(54) **Method for amplifying RNA**

(30) Priority: 12.03.2001 JP 2001069130
(71) Applicant: NISSHINBO INDUSTRIES, INC., Chuo-ku, Tokyo (JP)
(72) Inventor: Ichihara, Tatsuo, Midori-ku, Chiba-shi, Chiba (JP); Moriya, Shogo, Midori-ku, Chiba-shi, Chiba (JP)
(74) Representative: Tombling, Adrian George

(57) **Abstract**

Poly(A)⁺ RNA extracted from a cell or a tissue is amplified by the following steps of:
(a) synthesizing a cDNA first strand by using RNA extracted from the cell or the tissue as a template and a first primer having an oligo-dT and a promoter sequence,
(b) joining a sequence to which a second primer can anneal to the 3' end of the cDNA first strand,
(c) synthesizing a cDNA second strand by using the cDNA first strand as a template and the second primer to obtain a double-stranded cDNA, and
(d) synthesizing RNA by using the double-stranded cDNA obtained in the step (c) and an RNA polymerase corresponding to the promoter sequence.

SEQUENCE LISTING

<110> NISSHINBO INDUSTRIES, INC.

<120> METHOD FOR AMPLIFYING RNA

<130> OP1281

<140>
<141> 2001-03-

<150> JP 2001-69130
<151> 2002-03-12

<160> 1

<170> PatentIn Ver. 2.1

<210> 1
<211> 66
<212> DNA
<213> Artificial Sequence

<220>
<223> Description of Artificial Sequence: primer

<400> 1
tctagtcgac ggccagtgaa ttgtaatacg actcactata gggcgttttt tttttttttt 60
tttttt 66

## Description

### BACKGROUND OF THE INVENTION

### Field of the Invention

The present invention relates to a method and kit concerning amplification of RNA. More precisely, it relates to a method and kit used for amplification of RNA molecules, which are objects of analysis of information concerning gene expression frequency, researches in molecular biology and for forth. Furthermore, it relates to primers and RNA templates used for the kit and methods for producing them.

### Description of the Related Art

In order to analyze gene expression information, for example, expression frequency, it is required to measure mRNA, which is a transcript of a certain specific gene. Further, in order to identify of which gene a transcript is a transcription product, analysis is performed by using hybridization with a nucleic acid complementary to the sequence of the gene.

Although RNA is currently extracted from cells or tissues by various techniques, it is difficult to extract it in an intact form in any methods, since it is more likely to be decomposed compared with DNA. Therefore, obtained RNA consists of a mass of RNA molecules of the same molecular species with different lengths (RNA population), and analyses of RNA are conducted for DNA complementary to it. Furthermore, since cells are highly differentiated in higher organisms, it is required to precisely classify individual cells or cells in tissues and investigate which gene on DNA in those cells is activated. In order to analyze RNA obtained from such cells or tissues in a small amount, RNA must be amplified.

There are various nucleic acid amplification methods at present. As for nucleic acid amplification, there are the polymerase chain reaction (PCR, published), the NASBA method (nucleic acid sequence-based amplification, published) and so forth. Significance of these amplification methods in researches of molecular biology resides in increasing numbers of molecules of a small amount of DNA or RNA. If numbers of DNA or RNA molecules are increased, it becomes possible to detect them by various techniques as objects of researches. Therefore, great benefit can be obtained by amplifying a trace amount of DNA or RNA.

In the PCR amplification method, two specific sequences are selected from a sequence existing in a gene to be amplified or sequences of the both end portions thereof to prepare oligomer DNAs for the specific sequences. One oligomer DNA has the same sequence as one of the specific sequences, and the other has a sequence complementary to the other specific sequence. The prepared oligomers are used as primers, and they are annealed to a template DNA and then synthesis of a complementary chain is started. The PCR reaction consists of three steps of dissociation of template DNA, annealing of primers to the dissociated single-stranded template DNA and synthesis of complementary strand, and a cycle consisting of these three steps is repeated to amplify DNA. If the cycle is repeated n times, DNA will be finally amplified by 2n times. Usually, a heat resistant DNA synthetase is used so that the enzyme should not be inactivated in each cycle.

However, since RNA cannot be used as a direct template in the PCR method, a double-stranded DNA must be once synthesized by using RNA as a template and PCR amplification must be conducted by using this double-stranded DNA as a template (reverse transcription PCR). Further, since the amplified DNA is limited to DNA of a certain length corresponding to the setting positions of primers, there cannot be synthesized DNA corresponding to RNA of various lengths and sequences.

In the NASBA method, a specific primer having the T7 promoter sequence is annealed to RNA, and a first complementary DNA is synthesized by using a reverse transcriptase. After decomposing the RNA with RNase H, a second DNA is synthesized by using the first complementary DNA as a template and a second primer. The obtained double-stranded DNA consisting of the first strand and the second strand is used as a template and amplified through transcription into RNA by using a RNA polymerase. Further, a second primer is annealed to the amplified RNA, and a cycle consisting of the reverse transcription reaction, RNA decomposition, synthesis of complementary strand using the first primer and transcription with RNA polymerase is repeated. Although it is possible to amplify RNA by this method, the second primer needs to be configured in the sequence to be amplified and therefore the length of RNA to be amplified will be limited. Therefore, among the mass of RNA molecular species used as a template, RNA molecules having a length that does not allow annealing of the first and the second primers are not amplified, and thus the method is unsuitable for analysis of gene expression frequency. Furthermore, in both of the reverse transcription PCR method and the NASBA method, RNA to be amplified is limited to RNA having a specific nucleotide sequence. Therefore, if the amplified RNA is used, expression information can be obtained only for the specific gene.

As described above, it is impossible to amplify a mass of RNA molecular species by the conventional methods while maintaining abundance ratios of individual RNA molecular species before amplification.

### SUMMARY OF THE INVENTION

When analysis of gene expression frequency information is performed, it is important to accurately determine activity status of each gene. In order to detect genes of from extremely small expression frequency to high expression frequency, the transcript of gene, mRNA, must be amplified. However, mRNA cannot be amplified with reflecting a population of RNA by the currently available nucleic acid amplification methods as described above.

To solve such a problem, the present invention aimed at providing a method and kit for amplifying mRNA molecules in an mRNA mass while substantially maintaining population in the mRNA mass.

The inventors of the present invention assiduously studied in order to achieve the aforementioned object. As a result, they found that poly(A)⁺ RNA could be amplified while maintaining quantitative proportions of individual molecular species of the poly(A)⁺ RNA by synthesizing a cDNA first strand by using poly(A)⁺ RNA as a template and a first primer having a promoter sequence, joining a sequence to which a second primer could anneal to the 3' end of the cDNA first strand, allowing the second primer to anneal to the cDNA first strand, synthesizing a cDNA second strand to obtain a double-stranded cDNA, and performing an RNA polymerase reaction by using the obtained double-stranded cDNA as a template to obtain a RNA-DNA duplex. Thus, they accomplished the present invention.

That is, the present invention provides the followings.
(1) A method for amplifying poly(A)⁺ RNA extracted from a cell or a tissue, which comprises the following steps of:
   (a) synthesizing cDNA first strand by using RNA extracted from the cell or the tissue as a template and a first primer having an oligo-dT and a promoter sequence,
   (b) joining a sequence to which a second primer can an anneal to the 3' end of the cDNA first strand,
   (c) synthesizing a cDNA second strand by using the cDNA as a template and the second primer to obtain a double-stranded cDNA, and
   (d) synthesizing RNA by using the double-stranded cDNA obtained in the step (c) and an RNA polymerase corresponding to the promoter sequence.
(2) The method for amplifying RNA according to the above (1), which further comprises a step of decomposing the template RNA after the step (a).
(3) The method for amplifying RNA according to the above (1), wherein, in the step (b), the second primer is a first homopolymer and a second homopolymer that can anneal to the first homopolymer is joined to the 3' end of cDNA by using a terminal transferase.
(4) The method for amplifying RNA according to the above (1), wherein the promoter sequence is selected from the group consisting of T7 promoter, T3 promoter and SP6 promoter.
(5) The method for amplifying RNA according to the above (1), which further comprises the following steps of:
   (e) synthesizing cDNA by using the RNA synthesized in the step (d) as a template and the first primer as a template to obtain a RNA-DNA duplex, and
   (f) synthesizing RNA by using the RNA-DNA duplex obtained in the step (e) and an RNA polymerase corresponding to the promoter sequence.
(6) A method for producing a template DNA for amplifying poly(A)⁺ RNA extracted from a cell or a tissue, which comprises the following steps of:
   (a) synthesizing cDNA first strand by using RNA extracted from the cell or the tissue as a template and a first primer having an oligo-dT and a promoter sequence,
   (b) joining a sequence to which a second primer can an anneal to the 3' end of the cDNA first strand, and
   (c) a step of synthesizing a cDNA second strand by using the cDNA as a template and the second primer to obtain the template DNA.
(7) A kit for amplifying transcription products of a gene while maintaining quantitative proportions of the transcription products, which comprises a first primer having an oligo-dT and a promoter sequence and a second primer that anneals to a sequence that can be joined to the 3' end of cDNA cDNA first strand synthesized by using RNA extracted from a cell or a tissue as a template and the first primer, and can serve as a primer for synthesis of a cDNA second strand.

### BRIEF EXPLANATION OF THE DRAWINGS

Fig. 1 shows an outline of the method of the present invention.

Fig. 2 is a photograph showing result of electrophoresis of cRNA amplified by the method of the present invention.

Fig. 3 shows results of hybridization for slide glass fixed with *Arabidopsis thaliana* gene using amplified cRNA.

Fig. 4 shows ratios of expression amounts of the gene calculated from the results shown in Fig. 3, and ratios of expression amounts of the gene measured by the Northern hybridization method using the amplified cRNA as a probe.

### PREFERRED EMBODIMENTS OF THE INVENTION

Hereafter, the present invention will be explained in detail.

The method of the present invention is a method for amplifying poly(A)⁺ RNA, i.e., a gene transcription product (mRNA) having a poly(A) tail at the 3' end, and comprises the following steps of:
(a) synthesizing cDNA first strand by using RNA extracted from a cell or a tissue as a template and a first primer having an oligo-dT and a promoter sequence,
(b) joining a sequence to which a second primer can an anneal to the 3' end of the cDNA first strand,
(c) synthesizing a cDNA second strand by using the cDNA as a template and the second primer to obtain a double-stranded cDNA, and
(d) synthesizing RNA by using the double-stranded cDNA obtained in the step (c) and an RNA polymerase corresponding to the promoter sequence.

Each step will be explained hereafter.

### (1) Step (a)

An RNA fraction is extracted from a cell or tissue that is an object from which expression information of a gene is to be obtained. As the RNA fraction, poly(A)⁺ RNA is preferred. Poly(A)⁺ RNA can be usually extracted by a method well known as a method for extracting mRNA in cDNA cloning etc., for example, deproteinization using a protein denaturing agent such as phenol and guanidine thiocyanate, or affinity chromatography using oligo-dT cellulose or poly(A) Sepharose (Amersham Pharmacia) and so forth. Further, a commercially available kit for extraction and purification of mRNA can also be used.

Synthesis of cDNA first strand is performed by using RNA which has been extracted as described above as a template and a first primer having an oligo-dT and a promoter sequence. This primer has a promoter sequence on the 5' end side and an oligo-dT sequence on the 3' end side. Although number of T (deoxythymidine) in the oligo-dT sequence is not particularly limited so long as the oligo-dT can anneal to a poly(A) region of the poly(A)⁺ RNA and cDNA synthesis is possible, it may be usually about 10 to 40, preferably about 15 to 25, more preferably about 18 to 21. The promoter sequence is not particularly limited so long as it allows transcription reaction in a cell free system. Examples of the promoter sequence include those usually used for in vitro transcription such as T7 promoter, T3 promoter and Sp6 promoter. The first primer may have another sequence, unless the transcription from the promoter sequence utilizing DNA synthesized from the first primer as a template is inhibited. The first primer can be synthesized by a synthesis method usually used for DNA synthesis. An example of specific sequence of the first primer is shown as SEQ ID NO: 1.

The poly(A)⁺ RNA and the first primer are mixed in a solution to allow annealing of the poly(A) tail of the poly(A)⁺ RNA and the oligo-dT of the first primer. This is attained by, for example, heating the aforementioned solution at about 60-70°C and then quenching it. Then, a reverse transcription reaction is performed by using a reverse transcriptase. This reaction is performed in the presence of substrates for DNA synthesis (dATP, dGTP, dCTP, dTTP).

After this step, the template RNA is usually degraded. In the present invention, although this step is not indispensable, it is preferable to degrade the template RNA before the following steps. While the degradation of RNA can be performed by alkali hydrolysis or an enzymatic method utilizing RNase H or the like, the method using an alkali is preferred, since the final amplification production is RNA.

### (2) Step (b)

A sequence to which the second primer can anneal is joined to the 3' end of the cDNA first strand obtained in the aforementioned step (a). The second primer is not particularly limited so long as the primer can anneal to a sequence that is joined to the 3' end of cDNA and can annealed with the second primer, and it can function as a primer for the synthesis of a cDNA second strand. A homopolymer may be used as the second primer. The nucleotides constituting the homopolymer may be any of adenine, thymine, guanine and cytosine. The number of the bases in the homopolymer is usually about 5-40.

On the other hand, the sequence to which the second primer can anneal may be a sequence complementary to the second primer. When the second primer is a homopolymer, the sequence that can anneal thereto may be a homopolymer composed of nucleotides that can pair with the nucleotides constituting the homopolymer of the second primer. For example, when the second primer is an oligo-dT, the sequence that can anneal thereto is an oligo-dA. The homopolymer to which the second primer can anneal usually has a length similar to that of the second primer.

The homopolymer can be added to cDNA by using a terminal transferase.

### (3) Step (c)

Then, a cDNA second strand is synthesized by using the cDNA first strand to which the sequence that can be annealed with the second primer has been joined at the 3' end obtained in the step (b) as a template and the second primer to obtain a double-stranded cDNA. This reaction can be performed by using a DNA synthetase that utilizes a single-stranded DNA as a template, for example, DNA-polymerase I, Klenow fragment of the same, T4 DNA polymerase, reverse transcriptase or the like.

The double-stranded cDNA obtained through the above steps can be used as a template for the poly(A)⁺ RNA amplification using an RNA polymerase.

### (4) Step (d)

By using the double-stranded cDNA synthesized in the aforementioned step (c), RNA is synthesized with an RNA polymerase corresponding to the aforementioned promoter sequence. For example, when the promoter sequence is T7 promoter, T7 RNA polymerase is used. By this reaction, poly(A)⁺ RNA is amplified.

### (5) Step (e)

This step and the step (f) mentioned later are optional steps.

cDNA is synthesized by using the RNA synthesized in the aforementioned step (d) as a template and the first primer as a template to obtain a RNA-DNA duplex. The amplified cRNA and the first primer are annealed and a single-stranded cDNA is synthesized by a reverse transcription reaction. This reaction can be performed in the same manner as the reaction in the step (a).

### (6) Step (f)

Then, RNA is synthesized by using the RNA-DNA duplex obtained in the step (e) and an RNA polymerase corresponding to the aforementioned promoter sequence. This reaction can be performed in the same manner as in the step (d).

In each of the aforementioned steps, it is preferable to add any of various RNase inhibitors in order to inhibit decomposition of RNA by RNase. Further, in order to separate reaction products from the reaction substrates in the reactions using reverse transcriptase, terminal transferase and DNA synthetase, a separation process such as membrane filtration and ethanol precipitation may be performed. Further, after each reaction, the enzyme may be inactivated by heating or the like.

The kit of the present invention is a kit comprising a first primer having an oligo-dT and a promoter sequence and a second primer that anneals to a sequence that can be joined to the 3' end of cDNA synthesized by using the first primer and RNA extracted from a cell or a tissue as a template, and can be a primer for synthesis of a cDNA second strand. The kit of the present invention may further contain enzymes such as reverse transcriptase, terminal transferase, DNA synthetase and RNA polymerase, substrates for DNA synthesis and RNA synthesis, buffers, RNA inhibitors, purification devices for separating enzymatic reaction products and substrates.

The method of the present invention enables amplification of transcription products of a gene while maintaining quantitative proportions of the transcription products. Therefore, RNA amplified by this method is useful for analysis of gene expression frequency information and so forth.

### EXAMPLES

Hereafter, the present invention will be explained more specifically with reference to the following examples.

### Example 1: Synthesis of template for RNA amplification and amplification of cRNA using template for RNA amplification

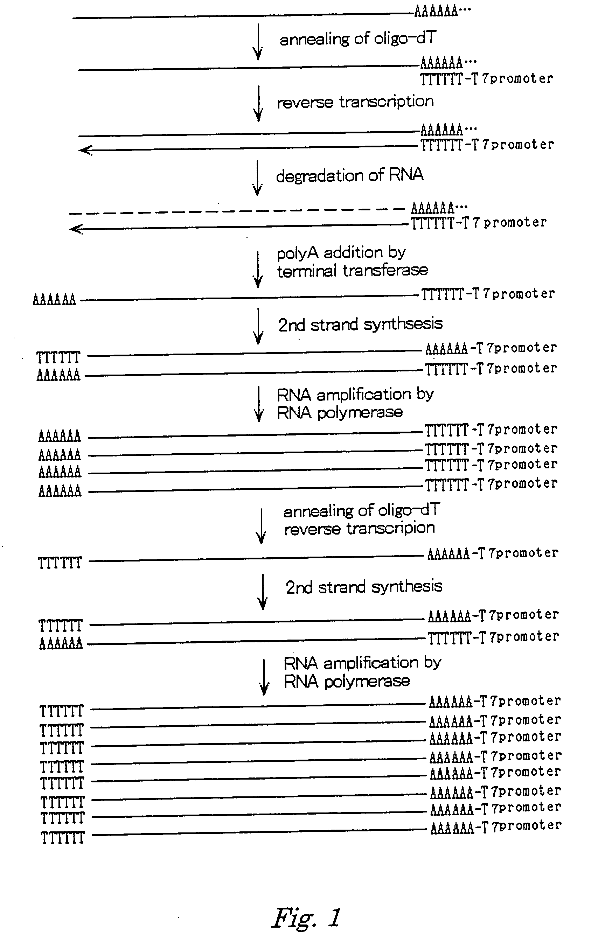

### (1) Synthesis of cDNA first strand with reverse transcriptase (Fig. 1 (a))

*Arabidopsis thaliana* mRNA in an amount of 2.0 µg was introduced into a 1.5-mL microtube, and added with 1 µL of 500 µg/mL of a first primer (SEQ ID NO: 1) and distilled water treated with diethyl pyrocarbonate to a total volume of 12 µL. The first primer had a structure consisting of the T7 promoter sequence ligated to 5' end of oligo-dT.

The aforementioned tube was heated at 70°C for 10 minutes and then rapidly cooled on ice. After the moisture adhered to the wall of the tube was spun down, 4 µL of a buffer (25 mM Tris-HCl (pH 8.3), 375 mM potassium chloride, 15 mM magnesium chloride), 2 µL of 0.1 M dithiothreitol solution and 1 µL of 10 mM dNTP solution (mixed solution of 10 mM dATP, 10 mM dGTP, 10 mM dCTP and 10 mM dTTP) were added to the reaction mixture in the tube. After the mixture was lightly stirred, it was heated at 42°C for 2 minutes. Then, the reaction mixture was added with 1 µL of 200 U/µL SUPERScriptII (Gibco BRL) and stirred lightly. The reaction mixture was left at 42°C for 50 minutes and then at 70°C for 15 minutes.

### (2) Degradation of template RNA (Fig. 1 (b))

The reaction solution utilizing reverse transcriptase was added with 1.5 µL of an alkaline solution (1 N NaOH, 20 mM EDTA) and left at 65°C for 10 minutes. The reaction solution was added with 270 µL of 10 mM Tris-HCl (pH 8.0) and 1.5 µL of 1 N hydrochloric acid. This solution was transferred to a centrifugal concentrator (Microcon YM-30, Millipore). The Microcon YM-30 was set on a centrifuge to perform centrifugation at 10000 rpm until the volume of the solution in a cup became 10 µL. After the solution was concentrated to a volume of about 10 µL, the cup was placed upside down in a fresh 1.5-mL tube and subjected to centrifugation at 3000 rpm for 5 minutes to collect the solution.

### (3) Addition of homopolymer to cDNA (Fig. 1 (c))

To 10 µL of the cDNA solution obtained in the above (2), 4 µL of a buffer (500 mM sodium cacodylate (pH 7.2), 5 mM cobalt chloride, 0.5 mM dithiothreitol), 5 µL of 100 mM dATP solution (Gibco BRL) and 1 µL of 10 U/µL terminal transferase (Gibco BRL) were added and left at 37°C for 1 hour to add an oligo-dA (5 to 35 bases) to the 3' end of cDNA.

### (4) Synthesis of second strand (template for RNA amplification) (Fig. 1 (d))

In an amount of 1 µL of 100 pmol/µL oligo-dT ((T)ₙ: n = 5-35) was added to 20 µL of the terminal transferase reaction solution of the above (3), heated at 95°C for 3 minutes, and then left on ice for 5 minutes to inactivate the terminal transferase. This solution was added with 4 µL of a buffer (100 mM Tris-HCl (pH 7.5), 70 mM magnesium chloride, 1 mM dithiothreitol), 4 µL of 0.2 mM dNTP (mixed solution of 0.2 mM dATP, 10 mM dGTP, 10 mM dCTP and 10 mM dTTP), 2 µL of 2 U/µL Klenow fragment (Takara Shuzo) and 9 µL of distilled water and allowed to react at 37°C for 2 hours. Then, the solution was left at 65°C for 5 minutes to inactivate the Klenow fragment.

### (5) Blunt-ending of termini of double-stranded cDNA

To 40 µL of the double-stranded cDNA solution obtained in the above (4), 4 µL of 3 U/µL T4 DNA polymerase (Gibco BRL) was added and left at 16°C for 5 minutes to blunt-end the termini of cDNA. This reaction mixture was added with 10 µL of 0.5 M EDTA, further added with about 40 µL of phenol:chloroform:isoamyl alcohol = 25:24:1, and stirred vigorously to denature the protein. This mixture was centrifuged at 15000 rpm for 2 minutes, and the upper layer was transferred to a new 1.5-mL microtube, added with the equal amount of chloroform and stirred vigorously. The mixture was centrifuged at 15000 rpm for 2 minutes, and the upper layer was transferred to another 1.5-mL microtube. Into this tube, 1/10 volume of 3 M sodium acetate (pH 5.2), 0.5 µL of glycogen (Roche Diagnostics) and 2.5-fold amount of 100% ethanol were introduced and left at -80°C for 20 minutes. Then, the mixture was centrifuged at 15000 rpm for 20 minutes and the supernatant was removed. The residue was added with 500 µL of 70% ethanol and centrifuged at 15000 rpm for 20 minutes, and the supernatant was removed. The precipitates were dissolved in 20 µL of distilled water to obtain a solution of template for RNA amplification.

### (6) Amplification of cRNA using template for RNA amplification (Fig. 1 (e))

The solution of template for RNA amplification of the above (5) was added with 6 µL of a buffer (200 mM Tris-HCl (pH 7.9), 30 mM magnesium chloride, 10 mM spermidine hydrochloride, 10 mM dithiothreitol) and the total volume was made 42 µL with distilled water. The solution was further added with 6 µL of rNTP solution (mixed solution of 75 mM ATP, 75 mM CTP, 75 mM GTP, 15 mM UTP and 15 mM fluorescein-labeled UTP (Fluorescein-UTP, Roche Diagnostics)), 6 µL of RNA polymerase (Ambion) and 1 µL of RNase inhibitor (Ambion) and allowed to react at 37°C for 2 hours.

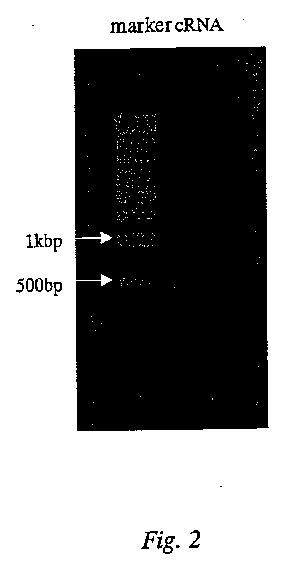

After the reaction, a part of the reaction mixture was taken and subjected to electrophoresis. The result is shown in Fig. 2.

### Example 2: Amplification of cRNA and hybridization using amplified cRNA

### (1) Amplification and purification of cRNA

mRNA of *Arabidopsis thaliana* exposed with ozone for 0 hour or 1 day was amplified by the method used in Example 1, respectively. The amplified RNA was labeled with fluorescein labeled-UTP (Fluorescein-UTP) in the case of the amplification of mRNA irradiated with ozone for 0 hour or with biotin-labeled UTP (Biotin-UTP, Roche Diagnostics) in the case of the amplification of mRNA irradiated with ozone for 1 day.

Each of these solutions was transferred to Microcon YM-30 for each. Each Microcon YM-30 was set on a centrifuge and concentrated at 10000 rpm until the volume of the solution in the cup became 2 µL. After each solution was concentrated to about 2 µL, the cup was set in a fresh 1.5-mL tube upside down and centrifuged at 3000 rpm for 5 minutes to collect the solution.

### (2) Preparation of slide glass on which gene of Arabidopsis thaliana is immobilized

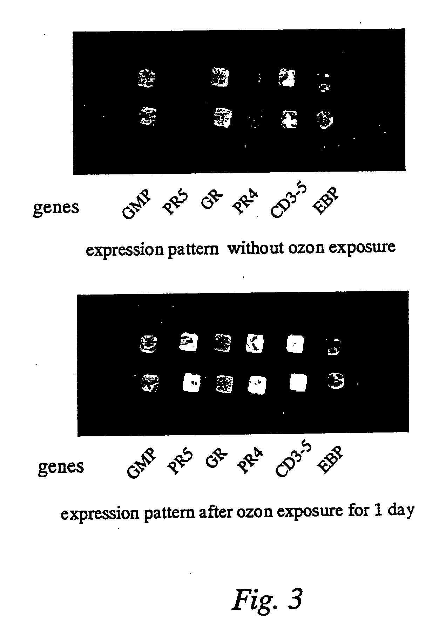

The genes shown in Fig. 3 were each dissolved in TE buffer (10 mM Tris-HCl (pH 7.2), 1 mM EDTA) at a concentration of 1 pmol/µL to form DNA solutions. Each of the DNA solutions was spotted on slide glass coated with carbodiimide resin by using a spotter (Pixsys 5000, Cartesian). The slide glass was put into a dryer and dried at 37°C for 15 minutes. Subsequently, the slide glass was immersed in Buffer A (0.2 M sodium chloride, 0.1 M Tris-HCl (pH 7.5), 0.05% Triton X-100) containing 3% BSA (bovine serum albumin) and then dried at 37°C for 15 minutes. Then, the slide glass was washed with TE buffer and dried at 37°C for 15 minutes.

### (3) Hybridization using cRNA

In an amount of 2 µL of each cRNA solution obtained by (1) and 8 µL of a hybridization solution (ExpressHyb, Clontech) were mixed and dropped onto the slide glass produced in (2) on which the gene of *Arabidopsis thaliana* was immobilized. Cover glass was placed on it and hybridization was allowed at 65°C for 12 hours. The cover glass was removed and the slide glass was immersed into 100 mL of a buffer (33.3 mM sodium chloride, 33.3 mM trisodium citrate, 0.1% sodium dodecylsulfate) and shaken at room temperature for 5 minutes. After this procedure was repeated twice, the slide glass was immersed into 100 mL of a buffer (3.3 mM sodium chloride, 3.3 mM trisodium citrate, 0.1% sodium dodecylsulfate) heated to 40°C and shaken at 40°C for 5 minutes. After this procedure was repeated twice, the slide glass was rinsed in 100 mL of a buffer (3.3 mM sodium chloride, 3.3 mM trisodium citrate).

### (4) Detection of hybridization signal

A BlockAce solution [1.24 g/L of tris(hydroxymethyl)aminomethane, 6.27 g/L of tris(hydroxymethyl)aminomethane hydrochloride, 8.77 g/L of sodium chloride and 40 g/L of BlockAce (Snow Brand Milk Products)] in an amount of 1 mL was dropped onto the slide glass and left at room temperature for 30 minutes. The BlockAce solution was removed and 500 µL of the BlockAce solution was dropped.

Then, 200 µg/µL streptavidin (Funakoshi) was dropped onto the slide glass that used the biotin-labeled cRNA and left at room temperature for 30 minutes. The solution was removed and the slide grass was washed with the BlockAce solution. Subsequently, 500 µL of the BlockAce solution and 25 µg/mL of biotinylated anti-streptavidin (Funakoshi) were dropped onto the slide glass and left at room temperature for 30 minutes. After the solution was removed and the slide glass was washed with the BlockAce solution, 500 µL of the BlockAce solution and 2.5 µg/mL Cy5-labeled streptavidin (Amersham Pharmacia) were dropped onto the slide glass and left at room temperature for 30 minutes.

On the other hand, onto the slide glass that used fluorescein-labeled cRNA, 4 µg/mL anti-fluorescein (mouse IgG, Roche Diagnostics) was dropped and left at room temperature for 30 minutes. The solution was removed and the slide glass was washed with the BlockAce solution. Then, 500 µL of the BlockAce solution and 25 µg/mL Cy3-labeled anti-mouse IgG (Cy3 anti-mouse IgG, Amersham Pharmacia) were dropped onto the slide glass and left at room temperature for 30 minutes.

The solutions were removed from each slide glass, and the slide glass was washed in 100 mL of a solution containing 1.24 g/L of tris(hydroxymethyl)aminomethane, 6.27 g/L of tris(hydroxymethyl)aminomethane hydrochloride and 8.77 g/L of sodium chloride. The slide glass was taken out from the solution and subjected to centrifugation at 1000 rpm for 2 minutes. Subsequently, fluorescence on the slide glass was detected by using a scanner (Scanarray 4000, GSI Lumonics).

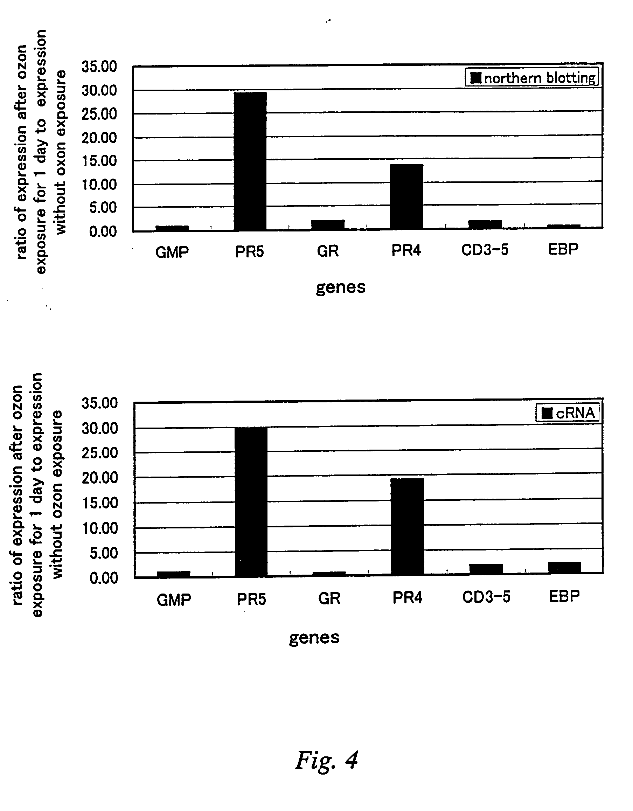

The results of the above are shown in Fig. 3. Further, ratios of expression amounts of the genes calculated from the results shown in Fig. 3 and ratios of the expression amounts measured by using the Northern hybridization method described later are shown in Fig. 4.

### (5) Northern hybridization

### (i) Labeling of probe

A probe was labeled by using Megaprime DNA Labelling System (Amersham). Each of the genes shown in Fig. 3 in an amount of 50 ng was added with pure water to a volume of 14 µL. It was added with 2.5 µL of a primer solution and thermally denatured at 100°C for 5 minutes. The mixture was returned to room temperature, added with 5 µL of labeling buffer, 2.5 µL of [³²P]dCTP and 1 µL of Klenow enzyme and incubated at 37°C for 10 minutes. After the reaction was terminated by adding 75 µL of TE buffer, the labeled probe was purified by using Sephadex G-50 (Pharmacia).

### (ii) Preparation of membrane for hybridizations

In an amount of 10 µL of total RNA, 1 x MOPS migration buffer (20 mM MOPS, 5 mM sodium acetate, 1 mM EDTA), 3.5 µL of formaldehyde and 10.0 µL formamide were mixed with pure water to obtain a total volume of 20 µL. This solution was heated at 65°C for 15 minutes and then quenched. The RNA was subjected to electrophoresis using 1% agarose gel (1% (W/V) agarose, 20 mM MOPS, 5 mM sodium acetate, 1 mM EDTA and 18% formaldehyde). The agarose gel on which the electrophoresis was performed was transferred to Hybond N⁺ (Amersham), dried at 80°C for 2 hours, and irradiated with an ultraviolet ray for 120 mMJ by using UV Stratalinker (Stratagene).

### (iii) Hybridization

The membrane prepared as described above was put into Hybripack (Gibco), added with 5 mL of a pre-hybridization solution (750 mM sodium chloride, 6.3 mM EDTA, 43 mM sodium dihydrogenphosphate, 50% (V/V) formamide, 0.1% (V/V) bovine serum albumin, 0.1% (V/V) Ficoll, 0.1% (V/V) polyvinylpyrrolidone and 0.5% SDS (W/V)), and sealed. Pre-hybridization was performed at 65°C for 1 hour. The labeled probe prepared in (i) was added and hybridization was allowed at 65°C for 12 hours.

### (iv) Post-hybridization

The membrane was taken out from Hybripack and washed with a primary washing solution (0.3 M sodium chloride, 17.3 mM sodium dihydrogenphosphate, 2.5 mM EDTA, 0.1% (W/V) SDS) at room temperature for 10 minutes. It was washed again with the primary washing solution at room temperature for 10 minutes. Then, the membrane was washed with a secondary washing solution (0.15 M sodium chloride, 8.7 mM sodium dihydrogenphosphate, 1.25 mM EDTA, 0.1% (W/V) SDS) at 65°C for 20 minutes. It was washed again with the secondary washing solution at 65°C for 20 minutes. Radioautography was performed by using the washed membrane. The results are shown in Fig. 3.

## Claims

1. A method for amplifying poly(A)⁺ RNA extracted from a cell or a tissue, which comprises the steps of:
(a) synthesizing a cDNA first strand by using RNA extracted from the cell or the tissue as a template and a first primer having an oligo-dT and a promoter sequence,
(b) joining a sequence to which a second primer can anneal to the 3' end of the cDNA first strand,
(c) synthesizing a cDNA second strand by using the cDNA first strand as a template and the second primer to obtain a double-stranded cDNA, and
(d) synthesizing RNA by using the double-stranded cDNA obtained in the step (c) and an RNA polymerase corresponding to the promoter sequence.

2. The method for amplifying RNA according to Claim 1, which further comprises a step of degrading the template RNA after the step (a).

3. The method for amplifying RNA according to Claim 1, wherein, in the step (b), the second primer is a first homopolymer and a second homopolymer that can anneal to the first homopolymer is joined to the 3' end of cDNA by using a terminal transferase.

4. The method for amplifying RNA according to Claim 1, wherein the promoter sequence is selected from the group consisting of T7 promoter, T3 promoter and SP6 promoter.

5. The method for amplifying RNA according to Claim 1, which further comprises the following steps of:
(e) synthesizing cDNA by using the RNA synthesized in the step (d) as a template and the first primer as a template to obtain a RNA-DNA duplex, and
(f) synthesizing RNA by using the RNA-DNA duplex obtained in the step (e) and an RNA polymerase corresponding to the promoter sequence.

6. A method for producing a template DNA for amplifying poly(A)⁺ RNA extracted from a cell or a tissue, which comprises the steps of:
(a) synthesizing cDNA first strand by using RNA extracted from the cell or the tissue as a template and a first primer having an oligo-dT and a promoter sequence,
(b) joining a sequence to which a second primer can an anneal to the 3' end of the cDNA first strand, and
(c) synthesizing a cDNA second strand by using the cDNA as a template and the second primer to obtain the template DNA.

7. A kit for amplifying transcription products of a gene while maintaining quantitative proportions of the transcription products, comprising a first primer and a second primer,
wherein the first primer has an oligo-dT and a promoter sequence and the second primer can anneal to a sequence that can be joined to the 3' end of cDNA first strand synthesized by using RNA extracted from a cell or a tissue as a template and the first primer, and can serve as a primer for synthesis of a cDNA second strand.
